# EUROPEAN PATENT SPECIFICATION

(11) **EP 3 578 559 B1**
(45) Date of publication and mention of the grant of the patent: **04.08.2021**
(21) Application number: 18020629.4
(22) Date of filing: 06.12.2018
(51) Int. Cl.: C07D 471/04, C09B 57/00, C09B 62/02

(54) **DYE FOR FLUORESCENT STAINING OF STRATUM CORNEUM, MANNER OF ITS SYNTHESIS AND APPLICATION IN MICROSCOPY, INCLUDING IDENTIFICATION OF ROUTES OF SUBSTANCES PENETRATION TO THE SKIN AND ASSESSMENT OF STRUCTURAL DISORDERS OF THIS LAYER**
FARBSTOFF ZUR FLUORESZENTEN FÄRBUNG VON STRATUM CORNEUM, VERFAHREN ZU DESSEN SYNTHESE UND ANWENDUNG IN DER MIKROSKOPIE, EINSCHLIESSLICH DER IDENTIFIZIERUNG VON ROUTEN VON SUBSTANZPENETRATIONEN AN DIE HAUT UND BEURTEILUNG VON STRUKTURELLEN STÖRUNGEN DIESER SCHICHT
COLORANT POUR COLORATION FLUORESCENTE DE LA COUCHE CORNÉE, MOYEN POUR SA SYNTHÈSE ET SON APPLICATION DANS LA MICROSCOPIE, Y COMPRIS L'IDENTIFICATION DE VOIES DE PÉNÉTRATION DE SUBSTANCES DANS LA PEAU ET L'ÉVALUATION DES TROUBLES DE STRUCTURE DE CETTE COUCHE

(30) Priority: 07.06.2018 PL 42584718
(43) Date of publication of application: 11.12.2019
(73) Proprietor: Gdanski Uniwersytet Medyczny, 80-210 Gdansk (PL)
(72) Inventor: SACZEWSKI, Jaroslaw Henryk, 80-398 Gdansk (PL); PIENKOWSKA, Krystyna, 80-119 Gdansk (PL); KRENCZKOWSKA, Dominika, 80-332 Gdansk (PL); CAL, Krzysztof, 80-522 Gdansk (PL); FEDOROWICZ, Joanna, 22-100 Chelm (PL)
(74) Representative: Godlewski, Piotr

(56) References cited:
- PL-B1- 223 740
- JAROSLAW SACZEWSKI ET AL: "The Tandem Mannich-Electrophilic Amination Reaction: a Versatile Platform for Fluorescent Probing and Labeling", CHEMISTRY - A EUROPEAN JOURNAL, vol. 19, no. 35, 26 August 2013 (2013-08-26), pages 11531-11535, XP055559984, DE ISSN: 0947-6539, DOI: 10.1002/chem.201302085
- FEDOROWICZ JOANNA ET AL: "Synthesis and fluorescence of dihydro-[1,2,4]triazolo[4,3-a]pyridin-2-iu m-carboxylates: An experimental and TD-DFT comparative study", DYES AND PIGMENTS, ELSEVIER APPLIED SCIENCE PUBLISHERS. BARKING, GB, vol. 161, 5 September 2018 (2018-09-05), pages 347-359, XP085510902, ISSN: 0143-7208, DOI: 10.1016/J.DYEPIG.2018.09.005

## Description

The subject of invention is a new fluorescent derivative of 1,2-dihydro-[1,2,4]triazolo[4,3-*a*]quinolin-2-ium-4-carboxylic acid, manner of its synthesis and application in fluorescent microscopy for dyeing the construction components of the *stratum corneum* targeted at assessing the barrier properties of human skin, structural disorders of *stratum corneum* under the influence of xenobiotics, observation of the effects of skin improvement pursuant to use of skin preparations and identification of the routes of penetration of substances to the skin. The invention is further referred to as SAFQ8MeAKT.

At present, an increased interest in the synthesis of new, innovative fluorescent dyes with new or innovative, physicochemical properties has been noted due to the necessity of visualizing cell structures, including skin in the course of realization of various research projects.

Human skin is an important research object within pharmaceutical, medical, biotechnological and biological studies. However, the level of research and the value of deducting on the basis of the obtained results strictly depends on the development of research techniques.

Diverse microscope techniques, including fluorescent ones, deemed as imaging techniques, are particularly appreciated. One must note at this point that usefulness of fluorescent microscope is strictly linked to the use of fluorescent dyes which ought to indicate specific affinity towards structures from which skin layers are construed: *stratum corneum, epidermis* and *dermis.*

Skin constitutes not only a significant route for the application of topical or systemic drugs, but it is also the most substantial barrier that protects the body against the harmful actions of external factors, be it mechanical, physical or biological but, above all, the chemical ones. In certain cases it however loses its capacity of being a protective barrier. It is particularly dangerous in case of toxic substances, strong-acting or such which have the capacity to cumulate in an organism. Thus, the assessment of skin barrier capacity (assessment of the degree of structural disorders) towards various xenobiotics (substances) is a crucial research objective, especially in the case of generally occurring substances in medical products designated for skin as well as cosmetics in products for body care. One ought to underline that the external layer, *stratum corneum,* is principally responsible for skin barrier capacity, characterized by a strict placement of corneocytes surrounded by a two-layer lipid, made of two adversely-structured layers of particles of lipid with non-polar, hydrophobic ends, turned inward to the layer and polar, hydrophilic groups turned outward. For this reason this layer is a particularly important object of research.

Fluorescent microscope imaging of *stratum corneum* provides unique information related to, for instance:
- identification of the construction components of this layer, which are formed by: corneocytes and the lipid matrix that surrounds them;
- assessment of penetration capacity of this layer by xenobiotics, including drugs and their permeation into deeper layers of skin (*epidermis* and *dermis*);
- Identification of the routes of penetration of substance to skin, the so called transport routes, which commonly include: transfolicular route - through hair follicle, sebaceous gland and sweat gland, transepidermal - perpendicularly to the cells of corneocytes and the lipid matrix as well as through the lipid matrix and canyons - surrounding the groups of corneocytes (so called clusters);
- understanding the processes (mechanisms) of the loss of barrier capacity of skin under the influence of operations of different substances;
- studying interactions of substances with the construction components of skin;
- assessment of the qualitative state of hair (colouration of the keratin protein);
- diagnostics of skin diseases;
- comparative analysis of disorder or improvement of the structure of this layer under the influence of drugs or conditioning agents on skin.

Fluorescent dyes *Safirinium-P* (P1 and P2) and *Safirinium-Q* (P4 and P5) (Scheme 1) and the method of their preparation have been disclosed in the patent description PL 223740 B. For the revealed structures the possibility of their use for fluorescent marking of bacterial spores has already been verified (Chem. Eur. J. 2013, 19, 11531 - 11535) and within proteomics as ionising markers within the tests carried out on protein hydrolysates (J. Pept. Sci. 2016, 22, (52), 99-100).

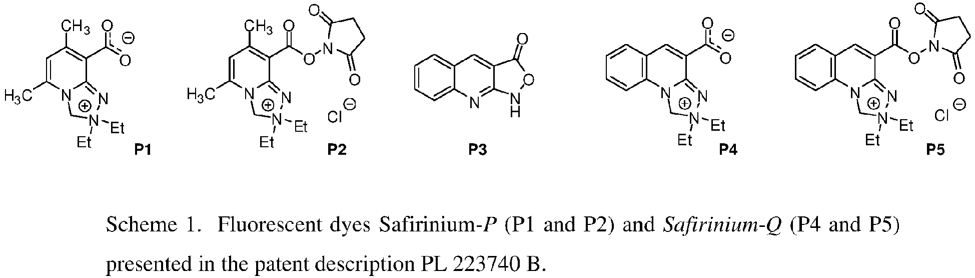

Physico-chemical properties of *Safirinium* dyes and fluorescent markers in the form of active esters with *N*-hydroxysuccinimide (NHS esters), such as: high quantum efficiency of fluorescence, significant Stokes' shifts, water solubility, chemical stability, resistance to photo-bleaching and lack of pH dependence of optic properties , predispose this group of compounds for the role of fluorescent dyes with potential applications.

Pursuant to the obtained results it was noted that without a targeted modification of the structure of *Safirinium* chromophore they have no specific affinity to the *stratum corneum* structures, which resulted in the lack of staining of the lipids inside the lipid matrix or corneocytes proteins (P1, P4), or in the case of interaction with the components of *stratum corneum,* very short duration of fluorescence - inside the lipid matrix and the canyons (P2, P5), in corneocytes (P2), in hair (P2).

In other words, on the basis of the conducted research these compounds were characterized in the following way:
P1 - too hydrophilic, which hinders diffusion to *stratum corneum,*
P2 - too hydrophilic, which hinders diffusion to *stratum corneum*
P3 - substrate in reaction, it is not a fluorescent dye,
P4 - too hydrophilic, which hinders diffusion to *stratum corneum,*(experimentally assigned partition coefficient logP = -1.90, which denotes octanol/water division ratio of 0,01),
P5 - too weakly lipophilic to stain the lipids of *stratum corneum* due to the impossibility of obtaining molecular dispersion in the lipids, conditioning sufficient dye solubility.

The most frequently applied commercial fluorescent dyes for the imaging of *stratum corneum* include:
1. Fluorescein (FLU) - demonstrates affinity to proteins, hydrophilic and lyophobic characteristics,
2. Sulforhodamines B (SRB) - demonstrates affinity to proteins, hydrophilic and lyophobic characteristics,
3. Rhodamine hexylester (RBHE) - demonstrates affinity to lipids, hydrophobic and lipophilic characteristics.

FLU and SRB stain corneocytes containing proteins, as well as enable observation of diffusion to *stratum corneum* of xenobiotics with lyophobic and hydrophilic characteristics, whilst RBHE dye stains only the lipids and enables observation of diffusion of lipophilic and hydrophobic xenobiotics to lipid matrix surrounding the corneocytes.

The objective of the hereby invention is modification and application in fluorescent microscopy of the new derivative of *Safirinium-Q* (structure P4 and P5), marked with acronym SAFQ8MeAKT with use in biomedical sciences for staining biological samples, including structures of *stratum corneum* for the assessment of barrier capacity of human skin, structural disorders of *stratum corneum* under the influence of xenobiotics, observation of the effects of improvement of the state of skin under the influence of preparations for skin and identification of routes of penetration of the substances to skin.

Subject of the invention is the derivative of 1,2-dihydro-[1,2,4]triazolo[4,3-*a*] quinolin-2-ium-4-carboxylic acid with formula 1.

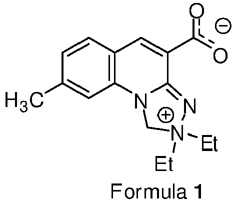

Subject of the invention is the derivative of 1,2-dihydro-[1,2,4]triazolo[4,3-a]quinolin-2-ium-4-carboxylic acid with formula 2.

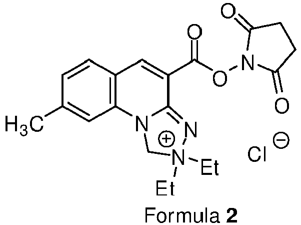

Subject of the invention is the method of obtaining the derivative with formula 1, defined above, where:
- 37% formaldehyde (146 µL, 0.90 mnol) is added while steering to the solution of 7-methylisoxazolo[3,4-*b*]quinolin-3(1*H*)-one (0.162 g, 0.81 mmol) and diethylamine (0.132 g, 1.80 mmol) in methanol,
- the reaction is carried out at room temperature within 10 minutes, after which the reaction mixture is evaporated under a reduced pressure, and the solid residue is washed with acetone (3 mL),
- obtained 2,2-diethyl-8-methyl-1,2-dihydro-[1,2,4]triazolo[4,3-*a*]quinolin-2-ium-4-carboxylate (**1**) is drained and dried under reduced pressure.

Subject of the invention is the method of obtaining the derivative with formula 2, defined above, where:
- 2,2-diethyl-8-methyl-1,2-dihydro-[1,2,4]triazolo[4,3-*a*]quinolin-2-ium-4-carboxylate (**1**) (0.30 g, 0.001 mol) dissolves in methanol (3 mL), after which the obtained solution is treated with the methanolic solution of hydrogen chloride till reaching acid reaction,
- then, the reaction mixture is evaporated under reduced pressure, while dry residue is treated with acetone (3 mL),
- the precipitated, drained and dried in vacuum desiccator product, *N,N'-*diisopropylcarbodiimide (DIC) (0.19 g, 0.0015 mmol) and N-hydroxysuccinimide (NHS) (0.23 g, 0.002 mmol) dissolve in dimethylformamide (DMF, 10 mL),
- reaction mixture is subjected ultrasounds for the period of 1 hour at room temperature, monitoring the reaction progress with the use of LC/MS,
- then DMF is evaporated under a reduced pressure while dry residue is washed with acetone (3 x 5 mL) and dried in vacuum desiccator.

Subject of the invention is fluorescent dye which is a derivative with formula 2, which stains the construction components of the layer of *stratum corneum:* corneocytes and lipid matrix, surrounded by corneocytes.

The dye which stains the canyons surrounding the groups of corneocytes which form the so called clusters.

The use of the derivative with formula 1, which has been defined above, displaying fluorescent action for preparation of fluorescent dye with general formula 2 from the derivative with general formula 1, having the use in biomedical sciences for staining biological samples, including structures of the layer of *stratum corneum.*

The use of the derivative with general formula 2, which was defined above, as a fluorescent dye, with the application in biomedical sciences for staining biological samples, including structures of *stratum corneum.*

The advantage of SAFQ8MeAKT dye is the ability to stain above all the lipids, as well as the protein of corneocytes of hair, that is keratin.

The obtained dye has a unique feature of staining within the *stratum corneum* the so called clusters which constitute, apart from the three generally described ways of transport of xenobiotics to skin: transfolicular - through hair follicle, sebaceous gland and sweat gland, transepidermal - perpendicular to the cells of corneocytes and lipid matrix and through lipid matrix, also significant, however described in subject literature in small extent, transdermal route of penetration of various substances to the organism. While staining the canyons one may observe the penetration of substance to skin, including active substances stemming from different formulations of medical products for skin or specific carriers, including liposomes. This dye may contribute to the development of research on barrier capacity of human skin towards various compounds (including medical or toxic, even with molecular weights larger than 500 Da), since penetration to skin may occur otherwise than it is generally considered, that is the diffusion process based on Fick's law. The canyons are lipid spaces between the clusters of corneocytes (clusters of tens of corneocytes) which are the subsequent degree of organization of specific layer of *stratum corneum,* existing for the skin barrier capacity. One must note that the canyons sometimes reach the depth where skin-epidermis border is located and a direct access to living dermis where blood vessels and lymphatic vessels are located. In the subject literature this route is still only slightly recognized at present.

The use of the new dye SAFQ8MeAKT enables observing the distribution of medical formulation or cosmetic formulation on the surface of *stratum corneum* or in the canyons located between the clusters of corneocytes.

The compound, according to the invention, has beneficial properties as a fluorescent dye: amphiphilic traits conditioning affinity both for the lipid structures and lyophobic ones, water-solubility, high quantum efficiency of fluorescence, large Stokes shift (95nm) and photo-optic durability.

The carried out tests indicated that the obtained post modification SAFQ8MeAKT compound, by way of the planned and optimized route of organic synthesis, fulfils not only the physico-chemical criterion (larger, optimal for diffusion of substance for skin lipophilicity than P4 and P5 dyes, covered by patent protection no. PL 223740), but also has essential for application (2,5-dioxopyrrolidin-1-yloxy)carbonylgroup, which conditions reactivity of the ester group towards nucleophiles.

As a result of further focused modification of the structure of chromophore *Safirinium-Q* (Patent PL 223740) new, 8-methyl derivative of *Safirinium Q* was obtained: 2,2-diethyl-8-methyl-1,2-dihydro-[1,2,4]triazolo[4,3-*a*]quinolin-2-ium-4-carboxylate and its active ester: 4-((2,5-dioxopyrrolidin-1-yloxy)carbonyl)-2,2-diethyl-8-methyl-1,2-dihydro-[1,2,4]triazolo[4,3-*a*]quinolin-2-ium chloride.

Reaction has been presented on Scheme 2.

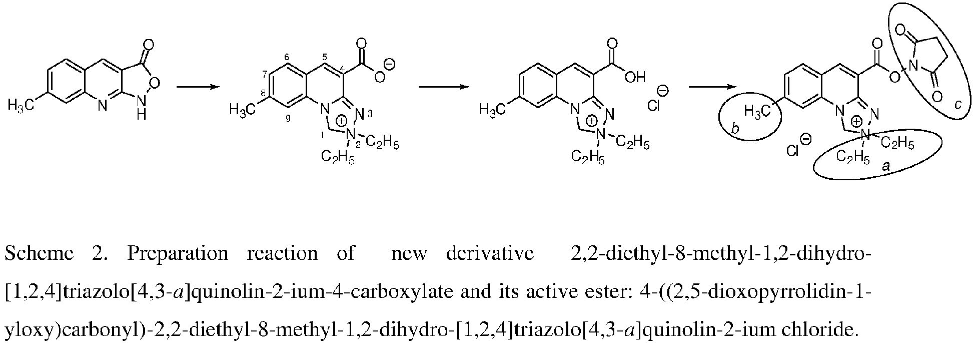

Modification leading to obtaining a new result covered three concepts:
a) introduction of diverse substituents at the N2 nitrogen atom (Scheme 2, part a) and/or
b) introduction of diverse substituents within the quinoline ring (Scheme 2 part b) and/or
c) introduction of (2,5-dioxopyrrolidin-1-yloxy)carbonyl group (Scheme 2, part c).

When planning to obtain new compounds the focus was also placed on the conservation of fluorescent properties (quinoline ring) and the optimal for diffusion to skin size of a particle below 500 Da. As previously noted, optimal lipophilicity (logP < 1) was crucial, so that the dye could penetrate *stratum corneum,* whilst not demonstrating a substantial capacity for deeper skin layers penetration (*epidermis* and *dermis*).

It was expected that the optimal lipophilicity (logP < 1) would enable staining , above all, the following structures:
- lipid from lipid matrix surrounding corneocytes,
- lipid from canyons surrounding corneocytes, so called clusters, as well as
- protein, i.e. keratin, filaggrin, involucrin, loricrin, trihohyalin, constituting the construction component of the cells of corneocytes.

Taking into consideration the most frequently applied fluorescent dyes which indicate lyophobic and hydrophilic traits (i.e. fluorescein - FLU or sulphorhodamine B -SRB) or lipophilic and hydrophobic (i.e. hexylorodamin B ester -RBHE) tests focused on obtaining a dye with amphiphilic properties. Amphiphilic traits of the compound indicate that it has affinity towards lipophilic, hydrophobic and also lyophobic, hydrophilic structures.

Amphiphilic traits increased the spectrum of use of the invention in comparison to the so far commercially applicable dyes (i.e. FLU, SRB, RBHE, Nile red), which allow specific (and through this more confined) skin imaging . Hence, introduction of amphiphilic traits to the dye allowed observation of the route of penetration into skin of both lipophilic as well as lyophobic compounds.

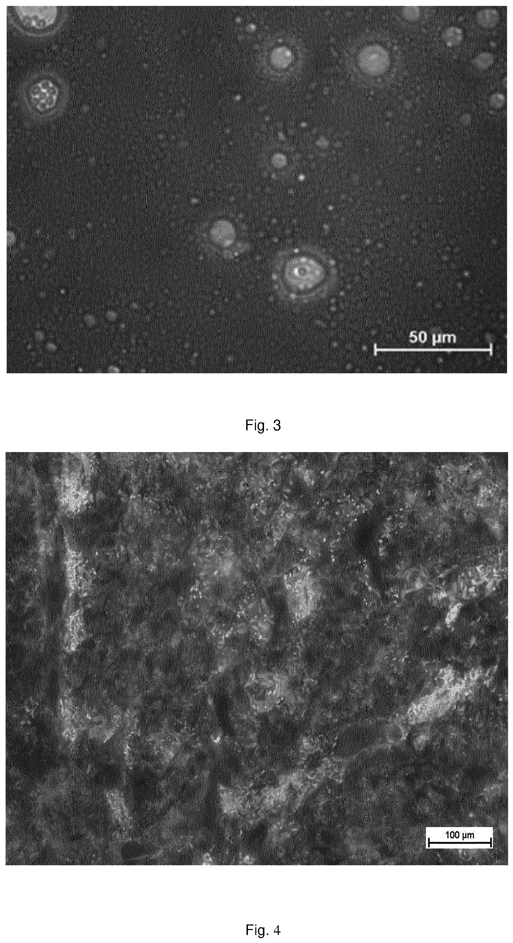

Amphiphilic nature of the invention with molecular mass Mₙ = 418,87 Da was documented on the basis of the designated logP value of = -0,43, as well as preparation of oil-in-water emulsion, in which fluorescent dye gathered on the border of phases water/octanol in the form of dark-blue ring (Fig. 4).

Carried out organic syntheses led to selecting optimal for application structure of SAFQ8MeAKT compound of the following features:
1. amphiphilic structure possessing groups showing affinity to both lipophilic and lyophobic structures;
2. molecular mass Mₙ=418,87 Da enabling overcoming the skin barrier and penetration of *stratum corneum.* Larger lengths of the alkyl chain at N2 nitrogen atom or quinoline ring would increase unbeneficial size of the molecular mass, which would prevent overcoming the skin barrier;
3. logP = -0,43 signifies that the ratio of the division of concentrations of this dye in the octanol/water composition amounted to 0,37. This value was indicated by means of an experiment. Modification of the particle enabled approx. 40 times greater lipophilicity than P4 dye, which impacted the ability to stain the lipids .
4. Introduction of (2,5-dioxopyrrolidin-1-yloxy)carbonyl group instead of carboxylic group allowed to obtain an active form of permanent fluorescence. Dyes with a free carbonyl group were characterized by fast extinguishing of fluorescence which was also visible in case of dyes with significant lipophilicity, obtained through introduction of longer alkyl chains (i.e. C₈H₁₇) at the N2 nitrogen atom.

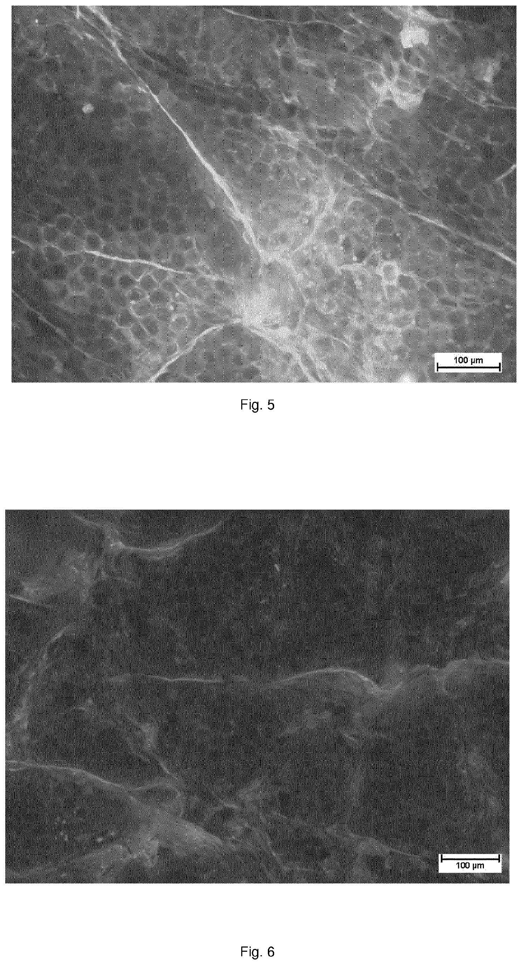

The performed studies indicated that all the selected elements of the obtained structure of SAFQ8MeAKT compound are equally valid to fulfil the criterion of applicability within biological research on human skin. It was observed that:
a) (2,5-Dioxopyrrolidin-1-yloxy)carbonyl group is very substantial in staining the structures of *stratum corneum,* impacting not only the lipophilicity compared to the analogues with a free carboxylic group, as well as increasing durability of florescence (prolonging duration of sample illumination).
   Analogue with a free carboxylic group at the quinoline ring was characterized by extinction of fluorescence after several seconds of microscope observation of the preparation.
b) (2,5-Dioxopyrrolidin-1-yloxy)carbonyl group connected to the pyridine ring (compound P2 from Patent PL 223740) did not guarantee permanent fluorescence. The compound quickly lost its fluorescent properties post staining of the preparation. This indicates an important role of the quinoline ring towards durability of fluorescence (conclusion based on comparison of points a and b).
c) Analogues with long alkyl chains (i.e. C₈H₁₇) at the N2 nitrogen atom, when compared to the invention, did not clearly stain the lipids, which may indicate that they did not diffuse to *stratum corneum* (too large molecule Mₙ=573,17 Da, too long alkyl chains and too large lipophilicity), but rather remained on the surface of the skin. They however were characterized by large durability of fluorescence.
d) Analogues with long alkyl chains (i.e. C₈H₁₇) at N2 nitrogen atom ,when compared to the invention analogue possessing free carboxylic group at the quinoline ring instead of ing (2,5-dioxopyrrolidin-1-yloxy)carbonylgroup, increased durability of fluorescence but did not stain the structures of *stratum corneum.* The dye was visible only on the surface of *stratum corneum* (Fig. 5; area. 10x). This indicates a significant role of (2,5-dioxopyrrolidin-1-yloxy)carbonyl group for staining of characteristic structures of *stratum corneum* - corneocytes and lipid matrix (conclusion based on comparison of points a, b, c and d).

Due to amphiphilic traits of the invention wider spectrum of application is noticed in tests on skin in comparison to the commercial fluorescent dyes.

Compound which is the subject of invention SAFQ8MeAKT has amphiphilic traits which grant affinity towards lipids of canyons surrounding the clusters of corneocytes, lipids of the lipid matrix (above all ceramides) surrounding single corneocytes as well as proteins (keratins) which are the construction component of corneocytes, enabling their simultaneous observation (Fig. 6 area 10x).

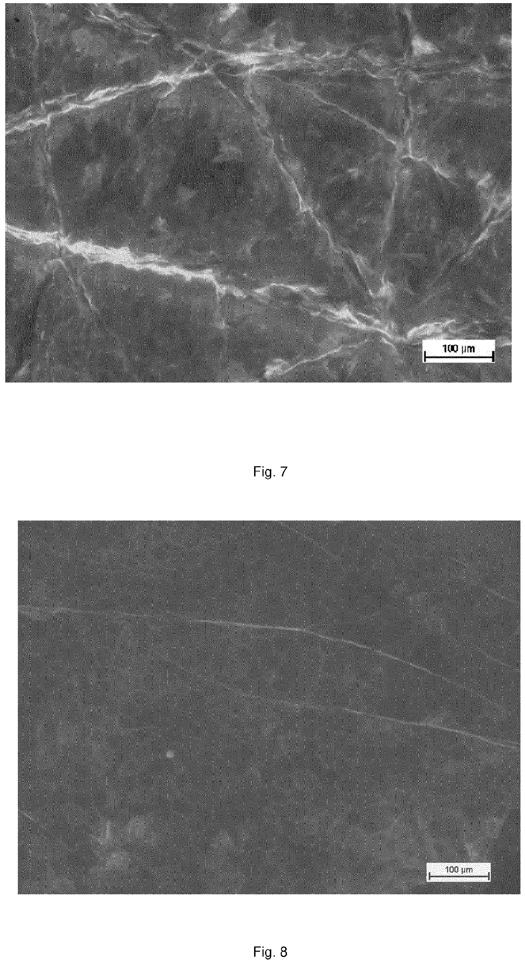

For comparison structures stained with a commercial dye RBHE with lipophilic traits was presented (Fig. 7 area 10x). Microscope image post applying RBHE dye is not as explicit (Fig. 7) as in the case of staining with SAFQ8MeAKT dye being the subject of invention (Fig. 6). In case of SAFQ8MeAKT the amphiphilic nature of the dye is visible through staining of the inside of corneocytes (Fig. 6 area 10x).

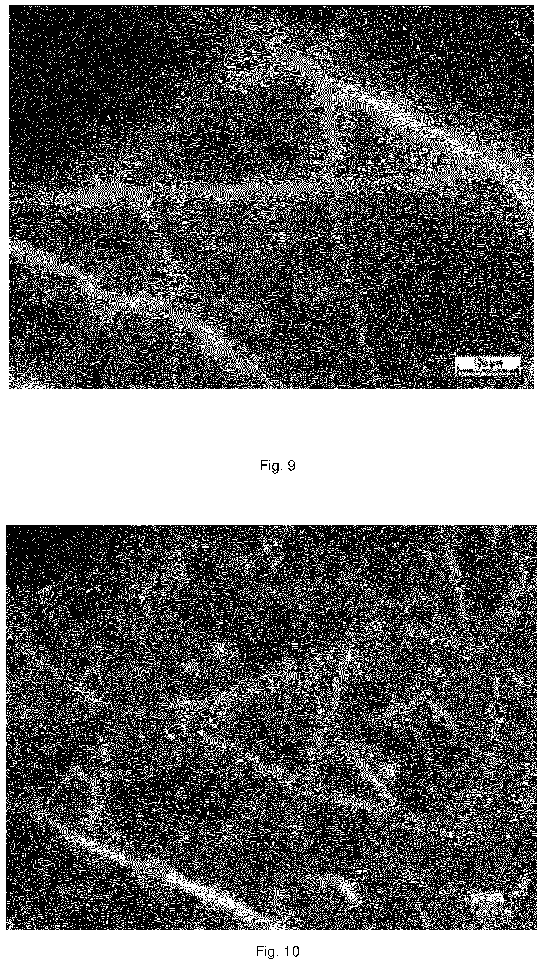

Fig. 8 and Fig. 9 present *stratum corneum* post application of water. Only in the case of invention SAFQ8MeAKT (Fig. 8) canyons are stained as an effect of dissolving the dye in water (hydrophilic part of amphiphilic structure), which indicates the possibility of observing the route of penetration into skin through cavities of canyons of not only lipophilic compounds (Fig. 6 area 10x), but also lyophobic (Fig. 8 area 10x). In the case of commercial RBHE canyons are not visible 9 area 10x).

These features caused application criterion fulfilment of SAFQ8MeAKT as fluorescent dye in fluorescent microscopy for visualization of *stratum corneum* and the route of penetration, including diffusion into skin of lipophilic substances (Fig. 6 area 10x) and lyophobic substances (Fig. 8 area 10x).

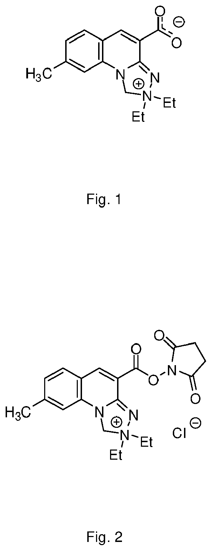

Derivative of 2,2-diethyl-8-methyl-1,2-dihydro-[1,2,4]triazolo[4,3-*a*]quinolin-2-ium-4-carboxylic acid, called SAFQ8MeAKT, obtained as a result of synthesis from derivative with chemical name 2,2-diethyl-8-methyl-1,2-dihydro-[1,2,4]triazolo[4,3-*a*]quinolin-2-ium-4-carboxylate (Fig. 1) is a new and innovative compound with fluorescent properties, dedicated as fluorescent dye for applications in fluorescent microscopy and confocal fluorescent microscopy for staining the construction components of *stratum corneum,* including corneocytes, lipid matrix and canyons.

Through analogy (on the basis of a new compound 2 as representative) it is possible also to obtain a new dye with fluorescent properties and a similar application as the above noted invention SAFQ8MeAKT as a result of synthesis with the use of derivative holding a chemical name of 2,2-diethyl-7-methyl-1,2-dihydro-[1,2,4]triazolo[4,3-*a*]quinolin-2-ium-4-carboxylate (Fig. 3).

In conclusion, the new compound SAFQ8MeAKT (Fig. 2) is a fluorescent dye which will be useful in fluorescent microscopy and confocal fluorescent microscopy for:
a) imaging of structures of *stratum corneum* of human skin:
   - corneocytes, built of proteins such as: keratin, filaggrin, involucrin, loricryn, trichohyalin,
   - lipid matrix, built from lipids such as: ceramids, cholesterol and its esters, free fatty acids, triglycerides and - canyons, built from lipids.
   The application of the subject invention in fluorescent microscopy or in confocal fluorescent microscopy is useful for evaluation of structural disorders or their lack in *stratum corneum,* responsible for skin barrier capacity.
b) establishing the route of penetration of xenobiotics to skin through lipid matrix, surrounding the corneocytes (transepidermal, intercellular route) or through less known in scientific literature canyons surrounding the clusters of corneocytes, as well as via intracellular or transfolicular route.
   Tests will be useful for the assessment of a degree of barrier capacity of human skin in terms of substances purposefully used in preparations or the undesired toxic substances.
c) study of the interaction of xenobiotics and the components of *stratum corneum* (lipids and proteins).

Tests will be useful for the assessment of the interaction of skin components with the components of dermal preparations (medical and cosmetic) or xenobiotics derived from the environment. The assessment of regenerative or destructive action of the formulation on skin through visualization of the shape of corneocytes, in comparison to their natural hexagonal or pentagonal shape.

Definitions applied above and within the description and patent claims have the following meaning:
**Biological sample** - refers to *stratum corneum,* hair.

**Application** - synonymously refers to application for use.

***Safirinium P*** - refers to the derivatives of 2,3-dihydro-[1,2,4]triazolo[4,3-*a*]pyridin-2-ium-8-carboxylic acid with the following formula:

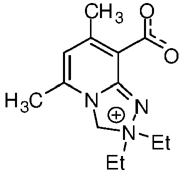

***Safirinium Q*** - refers to the derivatives of 1,2-dihydro-[1,2,4]triazolo[4,3-*a*]quinolin-2-ium-4-carboxylic acid with the following formula:

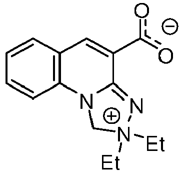

**SAFQ8MeAKT** - refers to 4-((2,5-dioxopyrrolidin-1-yloxy)carbonyl)-2,2-diethyl-8-methyl-1,2-dihydro-[1,2,4]triazolo[4,3-*a*]quinolin-2-ium chloride with a formula:

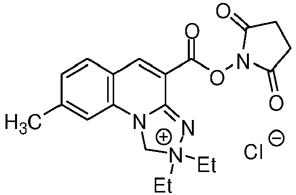

### Description of the figures:

**Fig.1** - 2,2-diethyl-8-methyl-1,2-dihydro-[1,2,4]triazolo[4,3-*a*]quinolin-2-ium-4-carboxylate
**Fig.2** - 4-((2,5-dioxopyrrolidin-1-yloxy)carbonyl)-2,2-diethyl-8-methyl-1,2-dihydro-[1,2,4]triazolo[4,3-*a*]quinolin-2-ium chloride
**Fig.3** - presents the microscopic image of water layer with visible drops of octanol phase, surrounded on the outside at the boundary of water-octanol phases of SAFQ8MeAKT dye; the effect of shaking water and octanol.
**Fig. 4** - presents the microscopic image of staining *stratum corneum* with analogues of the invention; 10 x lens zoom.
**Fig.5** - presents the microscopic image of staining *stratum corneum* with SAFQ8MeAKT dye after application of siloxane with a cyclic structure; 10 x lens zoom.
**Fig.6** - presents the microscopic image of staining *stratum corneum* with commercial dye RBHE after application of siloxane with a cyclic structure; 10 x lens zoom.
**Fig.7** - presents the microscopic image of staining *stratum corneum* with SAFQ8MeAKT dye after application of water; 10 x lens zoom.
**Fig.8** - presents the microscopic image of staining *stratum corneum* with commercial dye RBHE dye after application of water; 10 x lens zoom.
**Fig.9** - presents the microscopic image of staining *stratum corneum* with SAFQ8MeAKT dye after application of argan oil; 10 x lens zoom.
**Fig.10** - presents the microscopic image of staining *stratum corneum* with SAFQ8MeAKT dye after application of liposomes; 10 x lens zoom.
**Fig.11** - presents the microscopic image of staining *stratum corneum* with SRB dye; 10 x lens zoom.
**Fig.12** - presents the microscopic image of staining *stratum corneum* with SAFQ8MeAKT + SRB depigments mixture; 10 x lens zoom.

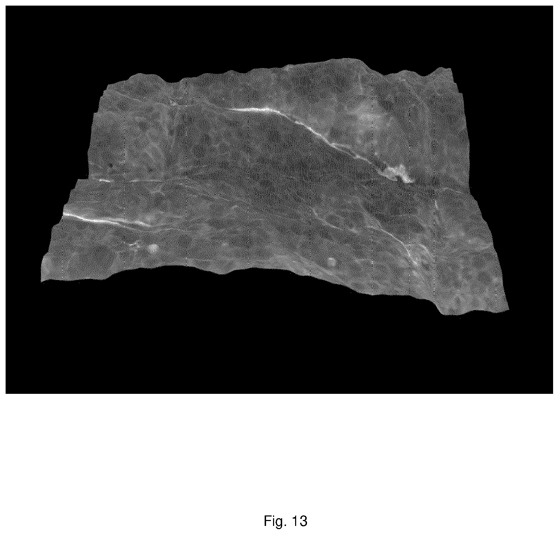

**Fig.13** - presents the microscopic image of staining *stratum corneum* with SAFQ8MeAKT + SRB depigments mixture; 3D image 10 x lens zoom.

The invention is illustrated by the following examples, which is not its limitation.

### Example 1

### Preparation of 2,2-diethyl-8-methyl-1,2-dihydro-[1,2,4]triazolo[4,3-a]quinolin-2-ium-4-carboxylate (1) Fig. 1.

To the solution of 7-methylisoxazolo[3,4-*b*]quinolin-3(1*H*)-one (0.162 g, 0.81 mmol) and diethylamine (0.132 g, 1.80 mmol) in methanol, 37% formaldehyde (146 µL, 0.90 mnol) is added during mixing. The reaction is performed at room temperature for 10 minutes, after which the reaction mixture is evaporated under reduced pressure, and to the dry residue acetone (3 mL) is added. The precipitated 2,2-diethyl-8-methyl-1,2-dihydro-[1,2,4]triazolo[4,3-*a*]quinolin-2-ium-4-carboxylate (**1**) is drained and dried under reduced pressure.
0.22 g (94%) of the product melting at 248-250°C is obtained. ¹H NMR (500 MHz, CD₃OD): δ = 1.41 (t, 6H, *J* = 7.1 Hz, CH₃), 2.51 (s, 3H, CH₃), 3.69-3.82 (m, 4H, CH₂), 5.77 (s, 2H, CH₂), 7.01 (s, 1H, CH), 7.18 (d, 1H, *J* = 7.8 Hz, CH), 7.64 (d, 1H, *J* = 7.8 Hz, CH), 8.19 (s, 1H, CH); ¹³C NMR (50 MHz, CD₃OD): δ = 7.2, 20.9, 61.4, 71.0, 113.6, 119.0, 120.8, 125.0, 129.9, 134.7, 141.3, 144.3, 155.4, 168.3; IR (KBr): 3422, 3015, 2984, 2960, 2923, 2853, 1623, 1337, 814, 749 cm⁻¹; MS (ESI) *m*/*z*: 286 [M+1]⁺; elementary analysis for (%) C₁₆H₁₉N₃O₂x0.5H₂O: C 65.29, H 6.85, N 14.28; found: C 65.09, H 6.95, N 13.93.

### Example 2

### Preparation of 4-((2,5-dioxopyrrolidin-1-yloxy)carbonyl)-2,2-diethyl-8-methyl-1,2-dihydro-[1,2,4]triazolo[4,3-a]quinolin-2-ium chloride (2) Fig. 2 - hereinafter referred to as SAFQ8MeAKT

2,2-Diethyl-8-methyl-1,2-dihydro-[1,2,4]triazolo[4,3-*a*]quinolin-2-ium-4-carboxylate (**1**) (0.30 g, 0.001 mol) is dissolved in methanol (3 mL), then the resulting solution is combined with methanolic hydrogen chloride to acid reaction. Next, the reaction mixture is evaporated under reduced pressure, and the dry residue is combined with acetone (3 mL), The precipitated, filtered and dried in vacuum desiccator product, *N,N*'-diisopropylcarbodiimide (DIC) (0.19 g, 0.0015 mmol) and *N-*hydroxysuccinimide (NHS) (0.23 g, 0.002 mmol) is dissolved in dimethylformamide (DMF, 10 mL), The reaction mixture is exposed to ultrasounds for 1 h at room temperature and the reaction progress is monitored with use of LC/MS, Then the DMF is evaporated under reduced pressure, and the dry residue is washed with acetone (3 x 5 mL) and dried in vacuum desiccator (Scheme 3).

0.275 g (65.6%) of 4-((2,5-dioxopyrrolidin-1-yloxy)carbonyl)-2,2-diethyl-8-methyl-1,2-dihydro-[1,2,4]triazolo[4,3-*a*]quinolin-2-ium chloride (**2**) melting at 166-168°C is obtained. IR (KBr): 3475, 3397, 2973, 1740, 1613, 1562, 1461, 1348, 1190, 1089, 1059, 822. MS (ESI) m/z: 383 [M]⁺.

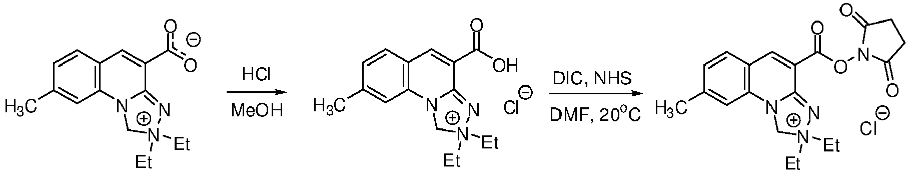

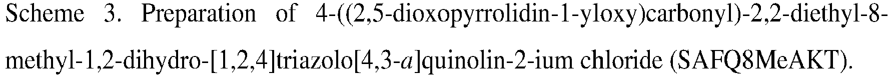

### Example 3

The use of the invention in fluorescent microscopy for imaging structures of *stratum corneum* of human skin was confirmed based on the sample application:
- lipophilic-octamethylcyclotetrasiloxane compound, D4 siloxane, with a cyclic structure - with a molecular weight of 297 Da and lipophilicity of logP = 4.45. D4 belongs to siloxanes, popularly known as silicones, which commonly are used in pharmaceutical and cosmetic preparations on the skin. Siloxanes (silicones) are present in app. 50% of cosmetics, Fig. 5. ;
- argan oil, which more often constitutes a component of the composition of preparations for body care and cosmetics (contains about 80% unsaturated fatty acids, with a predominance of omega-6), Fig. 9;
- liposomes with sizes up to 1 µm, obtained from sunflower oil used as transferors of active substances with lipophilic and hydrophilic traits used in cosmetic formulations. Liposomes are spherical bubbler sized built with water core and phospholipid membrane. The membranes of liposomes are composed of double phospholipid layers, between which water spaces are present. The membrane is compatible with a bilayer of lipid matrix, Fig. 10.

The composition of the liposomes suspension facilitating the penetration and absorption of fluorescent dye has been prepared according to the invention:
Lecithin 5-10 %
Penthylene glycol 1-3 %
Phenoxyethanol 0.5-1.0 %
Water to 100.0 %

Liposomes were prepared in accordance with the procedure: Penthylene glycol and phenoxyethanol are dissolved in water, then lecithin is added and gently stirred. It hydrates during 5-10 hours, homogenizes with ultrasounds 22 kHz for 10 minutes.

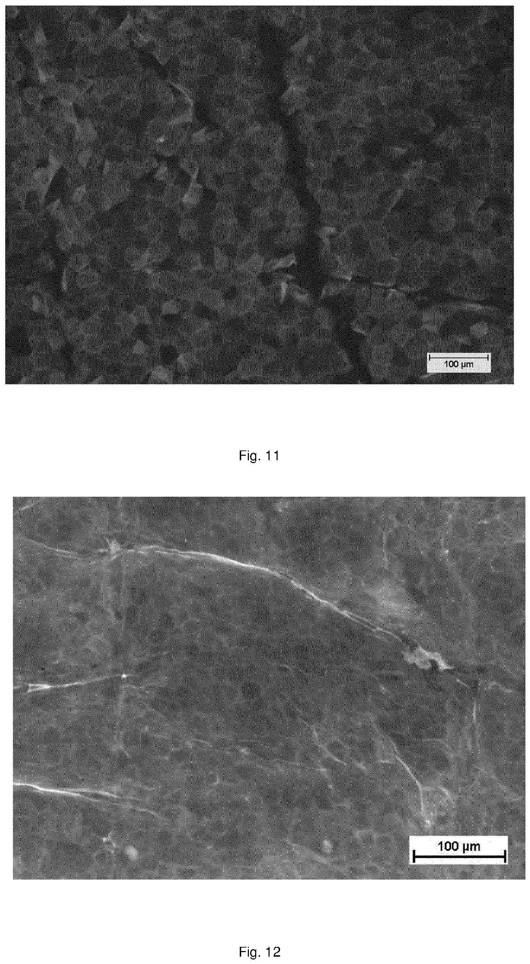

The executed study demonstrated:
a) Microscopic visualization of distribution (deployment) of test substances with lipophilic traits in lipid canyons surrounding corneocyte clusters of *stratum corneum.*
   In Fig. 5 - siloxane D4, Fig. 9 - argan oil, and in Fig. 10 - liposomes. The comparison of microscopic test samples stained with SAFQ8MeAKT dye with test samples stained with RBHE indicates that the invention with amphiphilic traits is more universal for a wider group of substances applied on the skin, what broadens the spectrum of skin barrier research. The commercial dye RBHE is not soluble in phospholipid liposomes, which are hydrophilic from the outside, therefore it is not possible to identify the way of liposomes penetration with this dye, which in turn is enabled by the invention SAFQ8MEAKT.
b) Implementation of the compound/dye SAFQ8MEAKT (blue coloured dye) to produce a two-component mixture with SRB dye (orange dye) of contradictive traits is an innovative utilization in the imaging of human skin. So far, the staining of the structures was carried out on the basis of one dye, for example, SRB (Fig. 11). This dye, with hydrophilic traits, noticeably stains corneocytes, constructed from protein filaments called keratin, but it does not dye the canyons surrounding the corneocyte clusters, which constitute an important route for the penetration of mostly lipophilic, but also hydrophilic substances. In Fig. 11 the canyons constitute the long undyed dark space.
   Due to the fact that SAFQ8MEAKT compound has amphiphilic traits and demonstrates blue fluorescence, it can be mixed with a hydrophilic SRB dye demonstrating orange fluorescence in order to contrast staining of specific structures of *stratum corneum.*
   So far, no studies with the use of dyes mixture for imaging *stratum corneum* structures have been published.
   This possibility may be a chance for a new quality of examining penetration of active and excipient substances with contradictive traits present in preparations on the skin.
   Application use of this mixture of dyes for staining the cell structures of *stratum corneum* was possible due to the contrast of fluorescence color of SAFQ8MeAKT (blue) and SRB (orange).
   Application of the excitation range in scope of λ = 330-380 nm allowed to obtain an image of fluorescence of both dyes. In accordance to Kasha's rule, the SRB can be excited with light of a higher energy than the range of the S1 absorption band, for SRB- characteristic absorption/emission is 565/590 nm, while for SAFQ8MeAKT the characteristic absorption/emission is 365/460 nm
c) The possibility of identification with use of the SAFQ8MeAKT dye the diffusion of lipophilic compounds to the skin through intercellular way through lipid matrix or lipids canyons surrounding clusters of corneocytes. Commercial dye RBHE does not stain canyons so vividly, as SAFQ8MeAKT does.
   It should be noted that the canyons can sometimes reach a depth, where is the dermal-epidermal border and access to live unprotected *dermis* cells, where the blood vessels and lymphatic vessels are located. In the literature, this path is still little recognized, but very important in the aspect of the ability of compounds to penetrate skin layers..
d) The possibility of identification of the diffusion of compounds of hydrophilic traits to the skin through intercellular way through lipid matrix or lipids canyons surrounding clusters of corneocytes with the use of the SAFQ8MeAKT dye. The commercial dye RBHE does not dye canyons in this case.
e) SAFQ8MeAKT compound will be applicable in the field of pharmacy (examining of preparations on the skin) and cosmetology (examining of cosmetics and body care products).
f) So far in fluorescent microscopy or confocal fluorescent microscopy for imaging of biological samples (for example, cells, tissues, cell lines of microorganisms) fluorescent dyes have been applied that demonstrate affinity to lipophilic and hydrophobic structures (e.g. sulforhodamine (B) hydroxyl ester; RBHE) or lyophobic and hydrophilic (such as fluorescein; FLU, sulforhodamine B; SRB).

The new compound SAFQ8MeAKT is a fluorescent dye that will be useful in fluorescent microscopy and confocal fluorescent microscopy for:
a) imaging *stratum corneum* structures of human skin:
   - the lipid matrix, that is built from lipids such as ceramides, cholesterol and its esters, free fatty acids, triglycerides and
   - corneocytes constructed from proteins such as keratin, filaggrin, involucrin, loricrin, trichohyalin.
   The study will be useful to assess the condition of the *stratum corneum,* responsible for barrier features of the skin.
b) establishment of penetration route of xenobiotics into the skin through the lipid matrix surrounding corneocytes (intercellular route) or less recognized in the scientific literature canyons surrounding clusters of corneocytes or by transcellular or transfolicular route.
   The study will be useful for assessing the extent of human skin barrier features in relation to the substances intentionally used in preparations or the adverse toxic substances.
c) interaction of xenobiotics with *stratum corneum* components (lipids and proteins).

The study will be useful for assessing the impact ingredients from dermal preparations (medical and cosmetic) or xenobiotics from the environment on the structures of the skin, including the degree of destructive action on the skin.

### Example 4

### The methodology of obtaining microscopic images:

To obtain microscopic images of *stratum corneum* in a fluorescent microscope (Nikon Eclipse 50, Japan; the mercury lamp; the filter for wavelength: 330-380 nm; software for data acquisition NIS Elements AR 3.2.) Standard methodology was applied. A fragment of human skin *ex vivo* (1 cm x 1 cm) was subject to *heat separation* procedure (temperature 60 °C; 30 seconds) in order to separate *epidermis* (including *stratum corneum*) from the *dermis.* Fragments of the *epidermis* were placed on a microscopic glass, fluorescent dyes were applied and then incubated in thermostat for 10 minutes at 37 °C. After this time, the excess of dyes was removed by the use of laboratory absorption blotting paper and images of microscopic magnification were recorded in 10 x magnification of the lens, using the exposure time of 15-600 ms.

## Claims

1. The derivative of 1,2-dihydro-[1,2,4]triazolo[4,3-*a*]quinolin-2-ium-4-carboxylic acid with formula **1.**

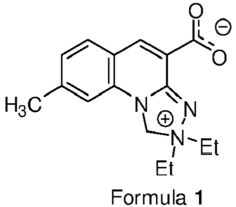

2. The derivative of 1,2-dihydro-[1,2,4]triazolo[4,3-*a*]quinolin-2-ium-4-carboxylic acid with formula **2.**

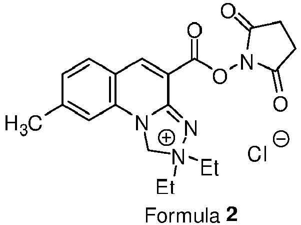

3. A method of obtaining the derivative of formula 1 defined in claim 1 **characterized by the following:**
- to the solution of 7-methylisoxazolo[3,4-*b*]quinolin-3(1*H*)-one (0.162 g, 0.81 mmol) and diethylamine (0.132 g, 1.80 mmol) in methanol, 37% formaldehyde (146 µL, 0.90 mmol) is added during mixing,
- the reaction is performed at room temperature during 10 minutes, after which the reaction mixture is evaporated under reduced pressure, and to the dry residue acetone (3 mL) is added,
- the precipitated 2,2-diethyl-8-methyl-1,2-dihydro-[1,2,4]triazolo[4,3-*a*]quinolin-2-ium-4-carboxylate (**1**) is drained and dried under reduced pressure.

4. A method of obtaining the derivative of formula 2 defined in claim 2, **characterized by the following:**
- 2,2-diethyl-8-methyl-1,2-dihydro-[1,2,4]triazolo[4,3-*a*]quinolin-2-ium-4-carboxylate (**1**) (0.30 g, 0.001 mol) is dissolved in methanol (3 mL), then the resulting solution is combined with methanolic hydrogen chloride to acid reaction,
- next, the reaction mixture is evaporated under reduced pressure, and the dry residue is treated with acetone (3 mL),
- the precipitated, filtered and dried in vacuum desiccator product, *N,N'-*diisopropylcarbodiimide (DIC) (0.19 g, 0.0015 mmol) and *N-*hydroxysuccinimide (NHS) (0.23 g, 0.002 mmol) is dissolved in dimethylformamide (DMF, 10 mL),
- the reaction mixture is exposed to ultrasounds for 1 h at room temperature and reaction progress is monitored with use of LC/MS,
- then the DMF is evaporated under reduced pressure, and the dry residue is washed with acetone (3 x 5 mL) and dried in a vacuum desiccator.

5. The use of the derivative according to claim 2, as fluorescent dye for staining the construction structures of the *stratum corneum*: corneocytes and the lipid matrix, surrounding corneocytes.

6. The use of the derivative according to claim 2, as fluorescent dye for staining canyons surrounding the groups of corneocytes creating so-called clusters.

7. The use of the derivative with general formula 1 that has been defined in claim 1, demonstrating fluorescent action for production of fluorescent dye of general formula 2 from the derivative of general formula 1, to the application of biomedical sciences for staining of biological samples, including structures of *stratum corneum.*

8. The use of the derivative of general formula 2 which was defined in claim 2 as a fluorescent dye with application use in biomedical sciences to staining of biological samples, including structures of *stratum corneum.*

## Patentansprüche

1. Derivat der 1,2-Dihydro-[1,2,4]triazol[4,3-*a*]chinolin-2-ium-4-carbonsäure mit der Formel **1.**

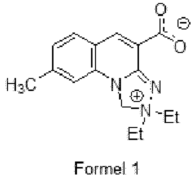

2. Derivat der 1,2-Dihydro-[1,2,4]triazol[4,3-*a*]chinolin-2-ium-4-carbonsäure mit der Formel **2.**

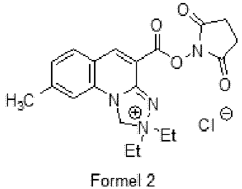

3. Verfahren zum Erhalten des Derivats der Formel 1, wie in Anspruch 1 definiert, **gekennzeichnet durch Folgendes:**
- zu der Lösung von 7-Methylisoxazol[3,4-*b*]chinolin-3(1*H*)-on (0,162 g, 0,81 mmol) und Diethylamin (0,132 g, 1,80 mmol) in Methanol wird 37% Formaldehyd (146 µL, 0,90 mmol) beim Mischen zugegeben,
- die Reaktion wird 10 Minuten bei Raumtemperatur durchgeführt, danach wird das Reaktionsgemisch unter vermindertem Druck verdampft und zu dem trockenen Rückstand wird Aceton (3 mL) zugegeben,
- das ausgefällte 2,2-Diethyl-8-methyl-1,2-dihydro-[1,2,4]triazol[4,3-a]chinolin-2-ium-4-carboxylat (**1**) wird abfiltriert und unter vermindertem Druck getrocknet.

4. Verfahren zum Erhalten des Derivats der Formel 2, wie in Anspruch 2 definiert, **gekennzeichnet durch Folgendes:**
- 2,2-Diethyl-8-methyl-1,2-dihydro-[1,2,4]triazol[4,3-*a*]chinolin-2-ium-4-carboxylat (**1**) (0,30 g, 0,001 mol) wird in Methanol (3 mL) gelöst, dann wird die resultierende Lösung mit methanolischem Chlorwasserstoff zur Säurereaktion vereinigt,
- danach wird das Reaktionsgemisch unter vermindertem Druck verdampft und der trockene Rückstand wird mit Aceton (3 mL) behandelt,
- das ausgefällte, filtrierte und im Vakuumexsikkator getrocknete Produkt *N,N'-*Diisopropylcarbodiimid (DIC) (0,19 g, 0,0015 mmol) und *N-*Hydroxysuccinimid (NHS) (0,23 g, 0,002 mmol) werden in Dimethylformamid (DMF, 10 mL) gelöst,
- das Reaktionsgemisch wird 1 h bei Raumtemperatur mit Ultraschall beaufschlagt und der Verlauf der Reaktion wird mittels LC/MS verfolgt,
- dann wird das DMF unter vermindertem Druck verdampft und der trockene Rückstand wird mit Aceton (3 x 5 mL) gewaschen und in einem Vakuumexsikkator getrocknet.

5. Verwendung des Derivats nach Anspruch 2 als Fluoreszenzfarbstoff zur Färbung der Konstruktionsstrukturen des *Stratum corneum*: Korneocyten und der die Korneocyten umgebenden Lipidmatrix.

6. Verwendung des Derivats nach Anspruch 2 als Fluoreszenzfarbstoff zur Färbung von Canyons, die die Gruppen von Korneozyten unter Bildung von sogenannten Clustern umgeben.

7. Verwendung des Derivats der allgemeinen Formel 1, wie in Anspruch 1 definiert, die fluoreszierende Aktivität zeigt, zur Herstellung des Fluoreszenzfarbstoff der allgemeinen Formel 2 aus dem Derivat der allgemeinen Formel 1 zur Anwendung in biomedizinischen Wissenschaften zur Färbung biologischer Proben, einschließlich Strukturen des *Stratum corneum.*

8. Verwendung des Derivats der allgemeinen Formel 2, wie in Anspruch 2 definiert, als Fluoreszenzfarbstoff zur Anwendung in biomedizinischen Wissenschaften zur Färbung biologischer Proben, einschließlich Strukturen des *Stratum corneum.*

## Revendications

1. Le dérivé de l'acide 1,2-dihydro-[1,2,4]triazolo[4,3-*a*]quinoléine-2-ium-4-carboxylique de formule **1.**

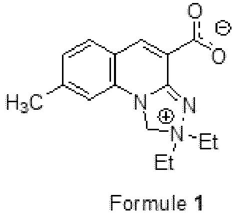

2. Le dérivé de l'acide 1,2-dihydro-[1,2,4]triazolo[4,3-*a*]quinoléine-2-ium-4-carboxylique de formule **2.**

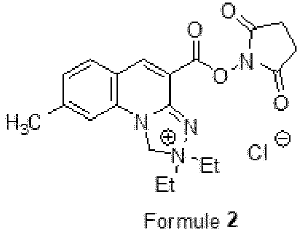

3. Procédé d'obtention du dérivé de formule 1 défini dans la revendication 1, **caractérisé par ce qui suit:**
- à la solution de 7-méthylisoxazolo[3,4-*b*]quinoléine-3(1*H*)-one (0,162 g, 0,81 mmol) et de diéthylamine (0,132 g, 1,80 mmol) dans du méthanol, du formaldéhyde à 37% (146 µl, 0,90 mmol) est ajouté au cours pendant le mélange,
- la réaction est effectuée à température ambiante pendant 10 minutes, après quoi le mélange réactionnel est évaporé sous pression réduite, et on ajoute au résidu sec de l'acétone (3 ml),
- le 2,2-diéthyl-8-méthyl-1,2-dihydro-[1,2,4]triazolo[4,3- a]quinoléine-2-ium-4-carboxylate précipité (**1**) est drainé et séché sous pression réduite.

4. Procédé d'obtention du dérivé de formule 2 défini dans la revendication 2, **caractérisé par ce qui suit:**
- Le 2,2-diéthyl-8-méthyl-1,2-dihydro-[1,2,4]triazolo[4,3-*a*]quinoléine-2-ium-4-carboxylate (**1**) (0,30 g, 0,001 mol) est dissous dans du méthanol (3 ml), puis la solution résultante est combinée avec du chlorure d'hydrogène méthanolique pour une réaction acide,
- ensuite, le mélange réactionnel est évaporé sous pression réduite et le résidu sec est traité à l'acétone (3 ml),
- le produit précipité, filtré et séché dans un dessiccateur sous vide, le *N,N'-*diisopropylcarbodiimide (DIC) (0,19 g, 0,0015 mmol) et le*N-*hydroxysuccinimide (NHS) (0,23 g, 0,002 mmol) est dissous dans du diméthylformamide (DMF, 10 ml),
- le mélange réactionnel est exposé aux ultrasons pendant 1 h à température ambiante et l'avancement de la réaction est surveillé à l'aide de LC/MS,
- puis le DMF est évaporé sous pression réduite, et le résidu sec est lavé avec de l'acétone (3 x 5 ml) et séché dans un dessiccateur sous vide.

5. Utilisation du dérivé selon la revendication 2, en tant que colorant fluorescent pour colorer les structures de construction du *stratum corneum*: cornéocytes et la matrice lipidique, entourant les cornéocytes.

6. Utilisation du dérivé selon la revendication 2, en tant que colorant fluorescent pour colorer des canyons entourant les groupes de cornéocytes créant ce que l'on appelle des amas.

7. Utilisation du dérivé de formule générale 1 qui a été définie dans la revendication 1, démontrant une action fluorescente pour la production d'un colorant fluorescent de formule générale 2 à partir du dérivé de formule générale 1, à l'application des sciences biomédicales pour colorer des échantillons biologiques, y compris des structures de *stratum corneum.*

8. Utilisation du dérivé de formule générale 2 qui a été définie dans la revendication 2 en tant que colorant fluorescent avec une application en sciences biomédicales à la coloration d'échantillons biologiques, y compris des structures de *stratum corneum.*
